# EUROPÄISCHE PATENTSCHRIFT

(11) **EP 4 003 244 B1**
(45) Veröffentlichungstag und Bekanntmachung des Hinweises auf die Patenterteilung: **22.11.2023**
(21) Anmeldenummer: 20744016.5
(22) Anmeldetag: 21.07.2020
(51) Int. Cl.: A61F 5/01

(54) **KNIEORTHESE**
KNEE ORTHOSIS
ORTHÈSE DE GENOU

(30) Priorität: 25.07.2019 DE 102019120181
(43) Veröffentlichungstag der Anmeldung: 01.06.2022
(73) Patentinhaber: Ottobock SE & Co. KGaA, 37115 Duderstadt (DE)
(72) Erfinder: KROLL-ORYWAHL, Olaf, 37154 Northeim (DE); SIEWERT, Gordon, 37083 Göttingen (DE)
(74) Vertreter: Gramm, Lins & Partner Patent- und Rechtsanwälte PartGmbB
(86) Internationale Anmeldenummer: PCT/EP2020/070531
(87) Internationale Veröffentlichungsnummer: WO 2021/013828

(56) Entgegenhaltungen:
- EP-A1- 2 959 868
- DE-A1- 3 433 843
- US-A- 3 575 166
- US-A- 4 681 097
- US-A- 4 940 045
- US-A1- 2014 316 317
- US-A1- 2015 080 777
- US-B1- 10 226 373

## Beschreibung

Die Erfindung betrifft eine Knieorthese mit einem Oberschenkelrahmen zum Anordnen an einem Oberschenkel und einen Unterschenkelrahmen zum Anordnen an einem Unterschenkel, wobei der Unterschenkelrahmen mittels wenigstens eines Gelenkes um eine erste Schwenkachse schwenkbar an dem Oberschenkelrahmen angeordnet ist, und einer Wadenpelotte zum Anlegen an eine Wade.

Derartige Knieorthesen sind aus dem Stand der Technik seit langem bekannt und werden insbesondere nach Operationen des hinteren Kreuzbandes verwendet, um die sogenannte "hintere Schublade" zu vermeiden. Insbesondere nach einer Ruptur des hinteren Kreuzbandes und der anschließenden Operation kann es dazu kommen, dass der Unterschenkel relativ zum Oberschenkel nach hinten rutscht, da es aufgrund der Verletzung zu einer Instabilität im Knie kommt. Dieses Verhalten ist seit langem bekannt und wird durch Knieorthese zu verhindern versucht.

Aus der EP 1 575 464 B1 ist beispielsweise eine gattungsgemäße Knieorthese bekannt, deren Wadenpelotte als Halbschale ausgebildet ist, die um die Wade herum angeordnet wird. Die Halbschale verfügt über ein knienahes Ende und über ein kniefernes Ende, die jeweils an einer Schiene angeordnet sind, wobei beide Schienen mit dem Schwenkgelenk zwischen dem Oberschenkelrahmen und dem Unterschenkelrahmen verbunden sind. Zwischen den beiden Schienen ist eine Feder angeordnet, durch die eine Kraft ausgeübt wird, durch die der Winkel zwischen den beiden Schienen verkleinert werden soll. Dadurch wird ein Drehmoment auf die als Halbschale ausgebildete Wadenpelotte ausgeübt, durch das der knienahe Anteil der Halbschale nach vorne gedrückt wird. Nachteilig ist jedoch, dass durch dieses Drehmoment eine als unangenehm empfundene Situation eintreten kann. Zudem kann die Halbschale insbesondere bei großer Flexion des Knies, also starker Beugung, den Kontakt zur Wade verlieren, sodass der gewünschte Effekt nicht oder nicht mehr vollständig auftritt.

Aus dem Stand der Technik sind zudem Knieorthese bekannt, die über einen Bowdenzug verfügen, durch den eine Beugung oder Flexion des Knies in einen aufgebrachten Druck umgesetzt wird, der von einer Wadenschale auf die Wade aufgebracht wird. Derartige Konstruktionen sind konstruktiv aufwendig und damit kostenintensiv und zudem fehleranfällig und wartungsintensiv. Zudem haben alle aus dem Stand der Technik bekannten Knieorthesen der hier beschriebenen Art den Nachteil, dass die durch die Wadenpelotte aufgebrachte Kraft nicht einstellbar ist und somit eine Reproduzierbarkeit nicht im gewünschten Maße gegeben ist.

Aus der US 4 940 045 A ist eine Knieorthese mit einem Oberschenkelrahmen und einem Unterschenkelrahmen bekannt, die gelenkig aneinander angeordnet sind. Ein Stützkissen wird seitlich an dem Unterschenkel angeordnet um die Kondylen zu stützen. Es wird eine seitliche Unterstützung für das versorgte Knie erzeugt.

Die US 2014/0316317 A1 befasst sich ebenfalls mit einer Knieorthese, die einen Oberschenkelrahmen und einen Unterschenkelrahmen aufweist, die gelenkig aneinander angeordnet sind. Durch ein elastisches Band kann eine Schwingunterstützung "swing assist" in Form einer Vorspannung erreicht werden. An der Wade liegt ein mit Luft gefülltes Kissen an. Durch die Vorspannung wird die Orthese in Richtung Extension vorgespann.

Die EP 2 959 868 A1 betrifft eine Knieorthese mit einem Oberschenkel- und einem Unterbeinanteil, die gelenkig miteinander verbunden sind. Der Unterbeinanteil weist dabei eine Wadenschale auf, die an die Wade des Nutzers gelegt wird. Über einen Kabelzug, der über Durchführungselemente an dem Unterbeinanteil von der gelenkigen Verbindung um die Wadenschale geführt wird, wird auf die Wade des Nutzers ein Druck in frontaler Richtung ausgeübt. Der Kabelzug ist hierbei über Spiralfedern in der gelenkigen Verbindung festgelegt. Die Konstruktion steht durch die Ausgestaltung des Kabelzugs bereits vor dem Anlegen unter Spannung

Der Erfindung liegt daher die Aufgabe zugrunde, eine konstruktiv einfache Knieorthese vorzuschlagen, durch die die aufgebrachte Kraft in komfortabler Weise für den Träger der Knieorthese aufgebracht wird und eine Reproduzierbarkeit der aufgebrachten Kraft erreicht wird.

Die Erfindung löst die gestellte Aufgabe durch eine Knieorthese gemäß dem Anspruch 1.

Die erfindungsgemäße Knieorthese verfügt folglich über einen Oberschenkelrahmen zum Anordnen an einem Oberschenkel. Dabei wird vorzugsweise wenigstens ein Gurt oder Riemen um den Oberschenkel herumgelegt, um den Oberschenkelrahmen am Oberschenkel zu fixieren. Der Rahmen selbst verfügt vorzugsweise über eine mediale Schiene und eine laterale Schiene, die beispielsweise durch den genannten Gurt miteinander verbunden sind. Die erfindungsgemäße Knieorthese verfügt zudem über einen Unterschenkelrahmen, der an einem Unterschenkel angeordnet werden kann. Auch dieser ist vorzugsweise über wenigstens einen Gurt oder Riemen am Unterschenkel befestigbar. Der Unterschenkelrahmen verfügt vorzugsweise über eine mediale Schiene und eine laterale Schiene, die miteinander verbunden sind. Der Unterschenkelrahmen und der Oberschenkelrahmen sind mittels wenigstens eines Gelenkes miteinander verbunden. Dabei handelt es sich vorzugsweise um ein Schwenkgelenk, das eine Verschwenkung des Oberschenkelrahmens relativ zum Unterschenkelrahmen um eine erste Schwenkachse herum erlaubt. Bevorzugt sind der Unterschenkelrahmen und der Unterschenkelrahmen durch zwei Gelenke ans Ende miteinander verbunden, deren Schwenkachsen übereinstimmen. Dabei ist bevorzugt ein Gelenk medial angeordnet und verbindet die mediale Schiene des Oberschenkelrahmens mit der medialen Schiene des Unterschenkelrahmens. Das zweite Gelenk ist bevorzugt lateral angeordnet und verbindet die laterale Schiene des Oberschenkelrahmens mit der lateralen Schiene des Unterschenkelrahmens.

Das wenigstens eine elastische Lagerungselement wirkt als elastischer Energiespeicher, der auch als mechanischer Energiespeicher bezeichnet wird. Beim Anlegen der Knieorthese wird das wenigstens eine elastische Lagerungselement gespannt. Dabei wird mechanische Energie, insbesondere Verformungsenergie in dem Lagerungselement gespeichert. Das wenigstens eine elastische Lagerungselement übt dann eine Kraft aus, durch die mittelbar oder unmittelbar die Wadenpelotte an die Wade des Trägers der Orthese gedrückt wird. Dies bedeutet insbesondere, dass das Lagerungselement mittelbar oder unmittelbar eine Kraft auf die Wadenpelotte ausübt, die in Richtung auf die Wade des Trägers wirkt. Dies kann auch eine Zugkraft sein, die auf die Wadenpelotte mittelbar oder unmittelbar ausgeübt wird.

Erfindungsgemäß verfügt die Knieorthese über einen Pelottenträger, der an dem Unterschenkelrahmen angeordnet ist. Der Pelottenträger kann beispielsweise ein Kunststoffelement, beispielsweise aus einem starren Kunststoff, oder ein Metallelement sein. Der Pelottenträger kann auch in Form eines vorzugsweise inelastischen aber flexiblen Elementes, beispielsweise eines textilen Gurtes, vorliegen. Bevorzugt ist der Pelottenträger an der medialen Schiene und der lateralen Schiene des Unterschenkelrahmens angeordnet. Er ist somit vorzugsweise an wenigstens zwei Stellen mit dem Unterschenkelrahmen verbunden. Dadurch wird eine erhöhte Stabilität erreicht.

An dem Pelottenträger ist das wenigstens eine elastisches Lagerungselement angeordnet. Es befindet sich vorzugsweise auf einer der Wade zugewandten Seite des Pelottenträgers und ist an diesem befestigt. Dabei kann das wenigstens eine elastische Lagerungselement lösbar oder unlösbar mit dem Pelottenträger verbunden sein. An dem wenigstens einen elastischen Lagerungselement ist die Wadenpelotte angeordnet, die der Wade zugewandt positioniert ist. Dabei handelt es sich beispielsweise um ein Polster oder Kissen, um ein angenehmes Tragegefühl für den Träger der Knieorthese zu erreichen. Wird die Knieorthese angelegt, wird der Oberschenkelrahmens am Oberschenkel und der Unterschenkelrahmen am Unterschenkel des Trägers angeordnet, sodass die erste Schwenkachse nahezu, bevorzugt jedoch möglichst exakt, mit der Schwenkachse des Knies des Trägers der Knieorthese übereinstimmt. Dabei wird die Wadenpelotte an der Wade des Trägers angeordnet und kommt mit dieser in Kontakt. Durch die geometrische Ausgestaltung und/oder Länge des Pelottenträgers wird erreicht, dass beim Anlegen der Knieorthese das wenigstens eine elastische Lagerungselement gespannt wird. Da es sich um ein elastisches Lagerungselement handelt, wird dadurch eine Rückstellkraft erzeugt, die der Spannung entgegenwirkt. Dadurch wird durch die Wadenpelotte eine Druckkraft auf die Wade des Trägers der Knieorthese ausgeübt.

Bevorzugt übt das wenigstens eine elastische Lagerungselement im gespannten Zustand eine nach frontal wirkende Kraft auf die Wadenpelotte aus, die durch die Wadenpelotte auf die Wade des Trägers übertragen wird. Anders als bei einigen Ausgestaltungen einer Knieorthese aus dem Stand der Technik wird daher in dieser Ausgestaltung kein Drehmoment auf die Wadenpelotte ausgeübt, das auf den Unterschenkel des Trägers übertragen wird, sondern das wenigstens eine elastische Lagerungselement erzeugt eine Kraft, die in eine einzige Richtung, vorzugsweise nach frontal, wirkt und somit den Unterschenkel nach vorn schiebt.

Vorzugsweise ist der Pelottenträger in mehreren unterschiedlichen Positionen und/oder Orientierungen relativ zu dem Unterschenkelrahmen an dem Unterschenkelrahmen befestigbar. Dies kann auf unterschiedliche Weise geschehen. Handelt es sich bei dem Pelottenträger beispielsweise um ein Kunststoff- oder Metallelement, kann dies eine Mehrzahl von Formschlusselementen aufweisen, wobei in diesem Fall am Unterschenkelrahmen korrespondierend ausgebildeter Formschlusselemente vorhanden sind. Dies kann beispielsweise in Form von Druckknöpfen geschehen, sodass der Pelottenträger in unterschiedlichen Positionen am Unterschenkelrahmen angeordnet werden kann. Insbesondere ist es auf diese Weise besonders einfach möglich, die wirksame Länge des Pelottenträger, also den Abstand zwischen den beiden Befestigungsstellen, an denen der Pelottenträger am Unterschenkelrahmen befestigt ist, einzustellen. Dabei wird dieser Abstand entlang des Pelottenträgers gemessen. Die Formschlusselemente können auch als Klettverschluss-Elemente vorhanden sein, wodurch eine stufenlose Veränderung der Position und/Orientierung des Pelottenträgers relativ zum Unterschenkelrahmen möglich wird.

Handelt es sich beim Pelottenträger um einen Gurt oder Riemen, kann dieser beispielsweise über eine Mehrzahl von Löchern aufweisen, die es ermöglichen, ihn an dafür vorgesehenen Schnallen, die beispielsweise am Unterschenkelrahmen angeordnet sind, zu befestigen. Durch die Wahl des jeweiligen Lochs, mittels dessen die Befestigung an der Schnalle erfolgt, lässt sich auch in dieser Ausgestaltung die wirksame Länge des Pelottenträgers einstellen.

Die wirksame Länge des Pelottenträgers ist dabei in diesen Ausgestaltungen entscheidend dafür, wie stark das wenigstens eine elastische Lagerungselement beim Anlegen der Knieorthese gespannt, bevorzugt komprimiert wird. Je kürzer die wirksame Länge ist, desto stärker wird das wenigstens eine elastische Lagerungselement gespannt, wenn die Knieorthese angelegt wird. Dadurch wird die von dem wenigstens einen elastischen Lagerungselement auf die Wadenpelotte und von dieser auf den Unterschenkel des Trägers übertragene Kraft verändert. Durch die Veränderung der Position und/oder der Orientierung des Pelottenträgers relativ zum Unterschenkelrahmen wird folglich die im angelegten Zustand auf die Wade und den Unterschenkel aufgebrachte Kraft einstellbar.

In einer bevorzugten Ausgestaltung ist die Position und/oder die Orientierung des Pelottenträgers relativ zu dem Unterschenkelrahmen stufenlos einstellbar und der Pelottenträger in jeder dieser stufenlos einstellbaren Positionen und/oder Orientierungen festlegbar. Dies wird auf besonders einfache Weise erreicht, wenn am Pelottenträger Klettverschlusses-Elemente und am Unterschenkelrahmen Klettverschluss-Gegenelemente angeordnet sind, sodass der Pelottenträger einfach vom Unterschenkelrahmen gelöst und in der gewünschten Position und/oder Orientierung wieder an ihm befestigt werden kann.

Vorzugsweise ist die Wadenpelotte lösbar an dem Pelottenträger angeordnet. Auch dies kann bevorzugt über Formschluss-Elemente geschehen, wie beispielsweise Druckknöpfe, Klettverschlusses-Elemente oder sonstige Elemente. Dadurch wird es möglich, die Wadenpelotte vom Pelottenträger zu entfernen und sie beispielsweise zu reinigen oder durch eine andere Wadenpelotte zu ersetzen. Dies kann beispielsweise sinnvoll sein, wenn die Wadenpelotte für die individuellen Anforderungen des Trägers der Knieorthese nicht geeignet ist, da sie beispielsweise zu oder zu klein ausgebildet ist oder ein anderer Polsterungsgrad gewünscht ist. Zudem ist es auf diese Weise möglich, die übrigen Bestandteile der Knieorthese wieder zu verwenden, sodass nicht jeder Patient eine eigene Knieorthese der hier beschriebenen Art benötigt. Dies ist von Vorteil, da die Knieorthese oftmals nicht über einen langen Zeitraum sondern nur für einen begrenzten Zeitraum beispielsweise nach einer chirurgischen Operation, verwendet wird.

Bevorzugt verfügt das wenigstens eine elastische Lagerungselement über ein Kompressionselement, das beispielsweise eine Druckfeder und/oder ein Bauteil aus einem elastischen Material ist. Dies kann beispielsweise aus einem Gummi oder einem Elastomer hergestellt sein, wobei die tatsächliche Elastizität des wenigstens einen elastischen Lagerungselement nicht nur vom Material des Kompressionselementes, sondern auch von dessen Geometrie abhängt. Vorzugsweise sind am Pelottenträger mehr als ein elastisches Lagerungselement angeordnet, wodurch erreicht wird, dass mehrere elastische Lagerungselement beim Anlegen der Knieorthese komprimiert werden und somit eine Gegenkraft auf die am Unterschenkel des Trägers anliegende Wadenpelotte ausüben. Auf diese Weise kann eine homogenere Kraftverteilung erreicht werden, wodurch der Tragekomfort und die therapeutische Wirksamkeit erhöht werden kann. Zudem wird die ohnehin bereits gute Autoadaption und auto-adaptive Anpassung der Form und Kontur der wenigstens einen Wadenpelotte an die Wade und den Unterschenkel des Trägers weiter verbessert.

Bevorzugt weist das wenigstens eine elastische Lagerungselement wenigstens ein Zugelement, vorzugsweise eine Zugfeder und/oder wenigstens einen elastischen Gurt auf. Durch wenigstens einen, bevorzugt zwei solche elastischen Gurte ist bevorzugt die Wadenpelotte an einem anderen Bauteil der Knieorthese, beispielsweise dem Unterschenkelrahmen angeordnet. Beim Anlegen wird das Lagerungselement gespannt, so dass Energie gespeichert wird. Dabei wird ein als elastischer Gurt ausgebildetes Lagerungselement gedehnt. Bevorzugt ist eine Dehnungsbegrenzung vorhanden, die beispielsweise in Form eines inelastischen Gurtes oder Gurtabschnittes ausgebildet ist, der parallel zu dem elastischen Gurt angeordnet ist. Dabei bedeutet "parallel" nicht in geometrischer Hinsicht parallel, sondern bezüglich der Wirksamkeit der beiden Gurte oder Gurtabschnitte parallel, insbesondere also nicht hintereinander oder in Serie angeordnet.

In einer bevorzugten Ausgestaltung ist die wenigstens eine Wadenpelotte um wenigstens eine zweite Schwenkachse schwenkbar an dem Pelottenträger angeordnet, wobei die zweite Schwenkachse vorzugsweise zumindest nahezu, besonders bevorzugt vollständig senkrecht auf der ersten Schwenkachse steht. Dies ermöglicht beispielsweise durch ein weiteres Schwenkgelenk oder Kugelgelenk die optimale Anpassung der Orientierung der Wadenpelotte an die individuellen Gegebenheiten der Wade und des Unterschenkels des Trägers. In optimaler Weise wird diese Anpassungsmöglichkeit erreicht, wenn mehrere miteinander gekoppelte Schwenkgelenke, ein Kardangelenk oder ein Kugelgelenk verwendet werden. Damit wird die Wadenpelotte in alle Richtungen schwenkbar am Pelottenträger über das wenigstens eine elastische Lagerungselement gelagert.

Vorzugsweise verfügt die Knieorthese über eine Messskala, an der eine Stärke der Kompression des wenigstens einen elastischen Lagerungselementes und/oder einer durch diese Spannung hervorgerufenen Kraft ablesbar ist. Auf diese Weise wird erreicht, dass die von dem gespannten wenigstens einen elastischen Lagerungselement hervorgerufene Kraft bestimmbar ist, sodass es möglich ist, sie auf einen gewünschten Wert einzustellen und auch zu einem späteren Zeitpunkt zu prüfen, ob die hervorgerufene Kraft die gewünschte Stärke weiterhin aufweist. Dies ist insbesondere durch die Verwendung der Messskala besonders einfach möglich, sodass dies auch durch den Patienten ohne die Hilfe eines Orthopädietechnikers geschehen kann. Dadurch wird der Aufwand bei der Verwendung der Knieorthese weiter reduziert, wodurch die Akzeptanz erhöht wird.

Mit Hilfe der beiliegenden Figuren werden nachfolgend einige Ausführungsbeispiele der vorliegenden Erfindung näher erläutert. Es zeigen:
- Figuren 1 und 2 -: schematische Seitenansichten zweier unterschiedlicher Ausführungsformen von Knieorthesen gemäß Ausführungsbeispielen der vorliegenden Erfindung,
- Figur 3 -: die schematische Darstellung einer Draufsicht auf eine Knieorthese gemäß einem Ausführungsbeispiel der vorliegenden Erfindung,
- Figuren 4 bis 6 -: vergrößerte Ausschnitte aus Knieorthesen gemäß weiterer Ausführungsbeispiele der vorliegenden Erfindung in einer Draufsicht,
- Figur 7 -: einen Ausschnitt aus einer Knieorthese gemäß einem weiteren Ausführungsbeispiel.
- Figuren 8 und 9 -: schematische Darstellungen einer weiteren Knieorthese gemäß einem Ausführungsbeispiel der vorliegenden Erfindung,
- Figur 10 -: ein Bauteil der Orthese aus den Figuren 8 und 9 und
- Figur 11 -: das Bauteil aus Figur 10 in einer Explosionsdarstellung.

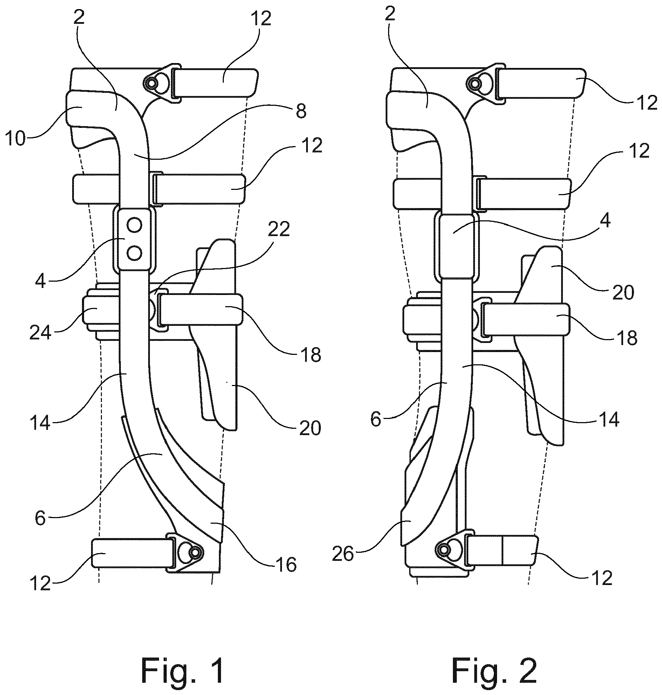

Figur 1 zeigt eine Knieorthese gemäß einem ersten Ausführungsbeispiel der vorliegenden Erfindung im angelegten Zustand. Sie verfügt über einen Oberschenkelrahmen 2, der über ein Gelenk 4 mit einem Unterschenkelrahmen 6 verbunden ist. Der Oberschenkelrahmen 2 verfügt über eine mediale Schiene 8 und eine in der Seitenansicht der Figur 1 nicht dargestellte laterale Schiene, die mit der medialen Schiene 8 über ein frontales Verbindungselement 10 verbunden ist. Im gezeigten Ausführungsbeispiel ist die mediale Schiene 8, die nicht dargestellt laterale Schiene sowie das frontale Verbindungselement 10 als einstückiges Bauteil ausgebildet. Über zwei Gurte 12 ist der Oberschenkelrahmen 2 am Oberschenkel des Trägers angeordnet.

Der Unterschenkelrahmen 6 verfügt ebenfalls über eine mediale Schiene 14 und eine in Figur 1 nicht dargestellte laterale Schiene. Die mediale Schiene 14 und die laterale Schiene sind über ein dorsales Verbindungselement 16 miteinander verbunden. Die mediale Schiene 14, die nicht dargestellte laterale Schiene und das dorsale Verbindungselement 16 sind in der Ausgestaltung gemäß Figur 1 einstückig ausgebildet. Der so gebildete Unterschenkelrahmen 6 wird über einen Gurt 12 am Unterschenkel fixiert.

Am Unterschenkelrahmen ist zudem ein Pelottenträger 18 angeordnet, an dem sich eine im gezeigten Ausführungsbeispiel als Halbschale ausgebildete Wadenpelotte 20 befindet. Dabei ist der Pelottenträger 18 über zwei Verbindungselemente 22, von denen in Figur 1 nur eines dargestellt ist, am Unterschenkelrahmen 6 angeordnet. An dieser Stelle befindet sich zudem ein weiterer Gurt 24, der für eine erhöhte Stabilität sorgt.

Figur 2 zeigt eine andere Ausgestaltung einer Knieorthese gemäß einem weiteren Ausführungsbeispiel der vorliegenden Erfindung. Auch diese verfügt über den Oberschenkelrahmen 2, der über zwei Gurte 12 am Oberschenkel des Trägers angeordnet ist und über ein Gelenk 4 mit einem Unterschenkelrahmen 6 verbunden ist. Anders als im in Figur 1 gezeigten Ausführungsbeispiel sind die mediale Schiene 14 und die nicht dargestellte laterale Schiene des Unterschenkelrahmens 6 über ein frontales Verbindungselement 26 miteinander verbunden. Der Unterschenkelrahmen 6 ist durch einen Gurt 12 am Unterschenkel festgelegt. Zudem befindet sich am Unterschenkelrahmen 6 wie bereits eben in Figur 1 gezeigten Ausführungsbeispiel der Pelottenträger 18 mit der sich daran befindenden Wadenpelotte 20.

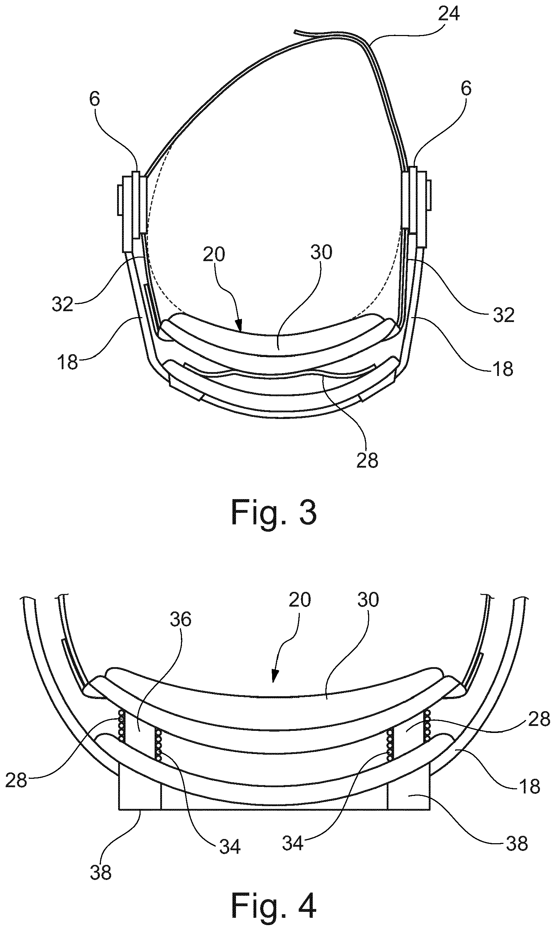

Figur 3 zeigt eine schematische Draufsicht auf eine Knieorthese gemäß einem Ausführungsbeispiel der vorliegenden Erfindung. Zur besseren Übersichtlichkeit ist der Oberschenkelrahmen weggelassen worden. Man erkennt den Pelottenträger 18, der an nur schematisch dargestellten Elementen des Unterschenkelrahmens 6 angeordnet ist. Er verläuft dorsal um das Bein des Trägers herum. Frontal befindet sich der Gurt 24, der vorzugsweise längenveränderlich ausgebildet ist und zum Anlegen der Knieorthese geöffnet werden kann.

Am Pelottenträger 18 ist schematisch dargestellt ein elastisches Lagerungselement 28, an dem sich die Wadenpelotte 20 befindet. Diese verfügt über ein Kissen 30, das vorzugsweise auswechselbar ist, um gereinigt oder durch ein anderes Kissen ersetzt werden zu können. Beim Anlegen der Knieorthese wird der Gurt 24 geöffnet und das Bein des Trägers in die Orthese eingeführt. Dabei wird das elastische Lagerungselement 28 komprimiert. Dies bedeutet insbesondere, dass die Wadenpelotte 20 in Richtung auf den Pelottenträger 18 verschoben wird, sodass ein Abstand zwischen der Wadenpelotte 20 und dem Pelottenträger 18 verkleinert wird. Da sich in diesem Zwischenraum das elastische Lagerungselement 28 befindet, wird es beim Anlegen der Knieorthese komprimiert.

Das wenigstens eine elastische Lagerungselement 28 ist im gezeigten Ausführungsbeispiel in Form einer Blattfeder ausgebildet und kann beispielsweise aus einem Metall oder einem Kunststoff bestehen. Aufgrund der Elastizität des elastischen Lagerungselementes 28 kommt es beim Komprimieren des Lagerungselementes 28 zu einer Gegenkraft, durch die die Wadenpelotte 20 gegen die Wade des Trägers gedrückt wird. Die Wadenpelotte 20 ist über Befestigungsgurte 32 im gezeigten Ausführungsbeispiel am Unterschenkelrahmen 6 angeordnet, um eine ausreichende Stabilität und mechanische Festigkeit zu gewährleisten.

Figur 4 zeigt einen vergrößerten Ausschnitt aus einer Draufsicht auf eine Knieorthese gemäß einem weiteren Ausführungsbeispiel. Man erkennt den Pelottenträger 18 sowie die Wadenpelotte 20 mit dem Kissen 30, die über im gezeigten Ausführungsbeispiel 2 elastische Lagerungselemente 28 am Pelottenträger 18 gelagert ist. Das jeweilige elastische Lagerungselement 28 umfasst eine elastisch komprimierbare Spiralfeder 34 und jeweils einen Führungsstift 36, der in einer Stiftaufnahme 38 verschiebbar gelagert ist. Beim Anlegen der Knieorthese wird wie bereits dargelegt, die Wadenpelotte 20 in Richtung auf den Pelottenträger 18 verschoben. Dabei werden die Führungsstifte 36 in die Stiftaufnahme 38 eingeschoben und gleichzeitig die Spiralfeder 34 komprimiert.

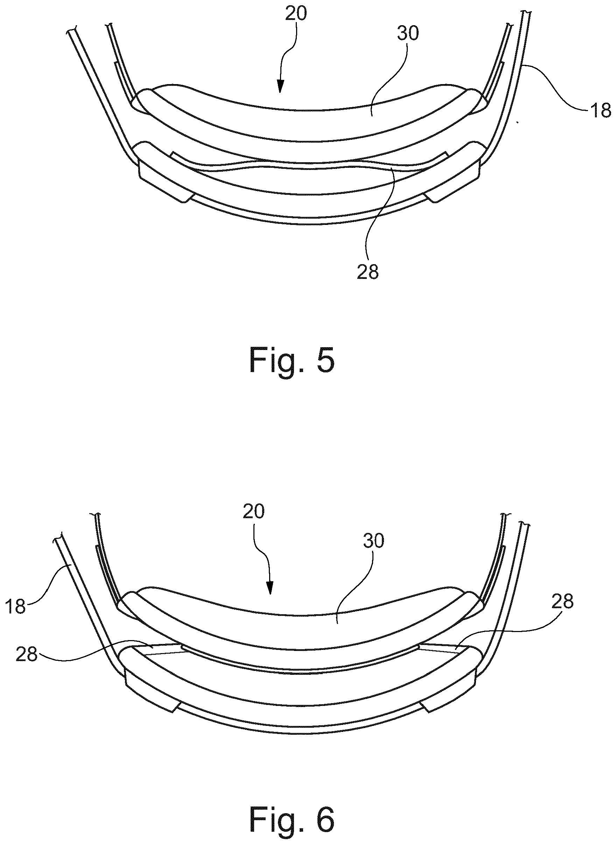

Die Figuren 5 und 6 zeigen zwei weitere Ausführungsformen, bei denen jeweils unterschiedliche elastische Lagerungselemente 28 vorhanden sind, über die die jeweilige Wadenpelotte 20 mit dem Kissen 30 am jeweiligen Pelottenträger 18 angeordnet ist. Figur 5 zeigt das elastische Lagerungselement 28, das bereits in Figur 3 dargestellt ist und eine Blattfeder aufweist. In Figur 6 sind zwei elastische Lagerungselemente 28 vorhanden, die als elastische Vorsprünge oder Laschen ausgebildet sind, an denen die Wadenpelotte angeordnet ist.

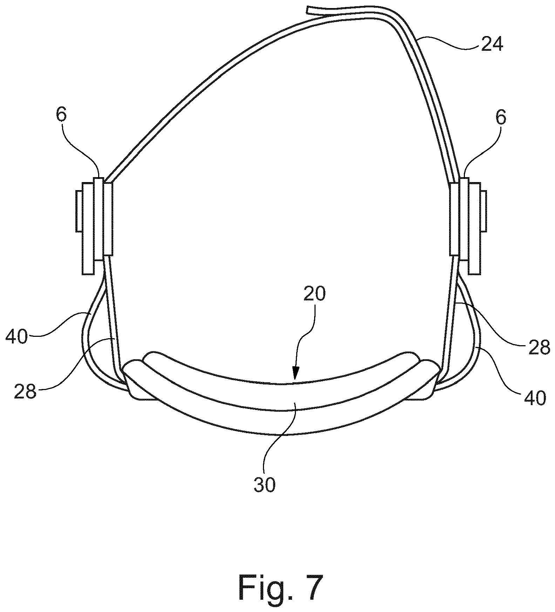

Figur 7 zeigt eine weitere Ausführungsform. Die Wadenpelotte 20 verfügt wie der über das Kissen 30 und ist am Unterschenkelrahmen 6 angeordnet. Im Unterschied zu den bisher gezeigten Ausführungsformen sind bei der in Figur 7 gezeigten Ausgestaltung die elastischen Lagerungselement 28 als Zugelement ausgebildet. Im gezeigten Ausführungsbeispiel sind die Lagerungselement 28 elastische Gurte, durch die die Wadenpelotte 20 am Unterschenkelrahmen 6 angeordnet ist. Beim Anlegen der Knieorthese werden die elastischen Lagerungselemente 28 gespannt, in der gezeigten Ausführungsform also gedehnt, sodass mechanische Energie gespeichert wird. Dadurch wird auf die Wadenpelotte 20 eine in Figur 7 nach oben wirkende Kraft ausgeübt, sodass die Wadenpelotte 20 mit einer nicht dargestellten Wade des Trägers in Kontakt bleibt. Parallel zu den Lagerungselement 28 sind zwei Dehnungsbegrenzer 40 dargestellt, die aus einem unelastischen Gurtabschnitt bestehen. Werden die Lagerungselement 28 zu stark gedehnt, erreichen Sie die Länge der in elastischen Gurtabschnitte der Dehnungsbegrenzer 40 und eine weitere Dehnung ist nicht möglich.

Auch in dieser Ausgestaltung wird durch den Gurt 24 die gezeigte Knieorthese am Unterschenkel des Trägers angeordnet.

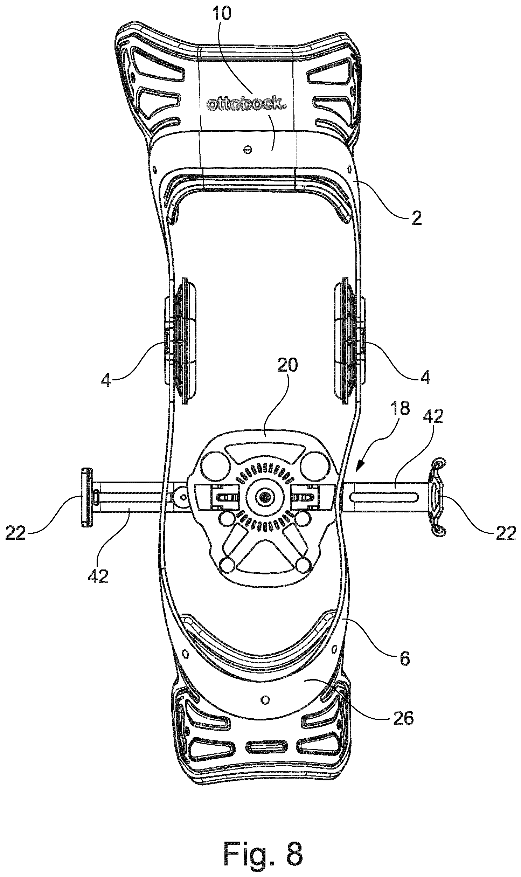

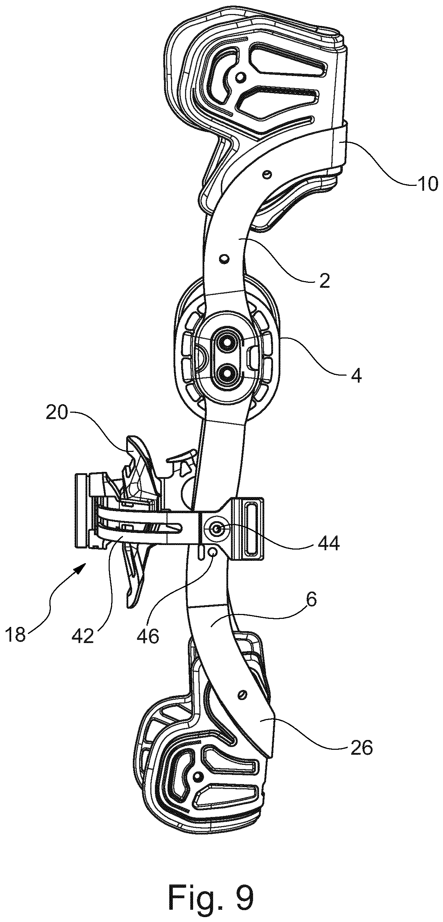

Figur 8 zeigt eine Frontalansicht einer Knieorthese gemäß einem weiteren Ausführungsbeispiel der vorliegenden Erfindung. Sie verfügt über zwei frontale Verbindungselemente 10, 26, von denen ein Teil des Oberschenkelrahmens 2 und das andere Teil des Unterschenkelrahmens 6 ist. Die Knieorthese verfügt über zwei Gelenke 4 über die der Unterschenkelrahmen 6 schwenkbar am Oberschenkelrahmen 2 angeordnet ist. Über einen Pelottenträger 18 ist die Wadenpelotte 20 am Unterschenkelrahmen 6 befestigt. An der Wadenpelotte 20 kann ein in Figur 8 und 9 nicht gezeigtes Kissen angeordnet werden, um eine Polsterung zu erreichen. Im gezeigten Ausführungsbeispiel verfügt der Pelottenträger 18 über zwei Arme 42, an deren jeweiligem Ende das Verbindungselement 22 angeordnet ist, mit dem der jeweilige Arm 42 am Unterschenkelrahmen 6 befestigt werden kann. In der in Figur 8 gezeigten Darstellung sind die Arme 42 nicht befestigt.

Figur 9 zeigt die Knieorthese aus Figur 8 in einer Seitenansicht von medial oder lateral. Der Oberschenkelrahmen 2 ist über das Gelenk 4 mit dem Unterschenkelrahmen 6 verbunden. Die beiden frontalen Verbindungselemente 10, 26 sind ausgebildet, am Oberschenkel und Unterschenkel angeordnet zu werden. Am Unterschenkelrahmen 6 erkennt man einen der Arme 42, die über einen Befestigungsstift 44 am Unterschenkelrahmen 6 befestigt sind, wobei der Befestigungsstift 44 in eines von im gezeigten Ausführungsbeispiel mehreren dafür vorgesehenen Löchern 46 hindurchgeführt wird. Der Arm 42 wird dabei, wie in Figur 9 dargestellt, gebogen. Er ist elastisch, zumindest jedoch flexibel ausgebildet. Am Pelottenträger 18, zudem die beiden Arme 42 gehören, ist die Wadenpelotte 20 angeordnet, die, wie bereits dargelegt, um ein nicht gezeigtes Kissen ergänzt werden kann. Sie ist gelenkig über ein elastisches Lagerungselement 28, das in Figur 9 nicht dargestellt ist, gelagert. Dieses elastische Element 28 kann beispielsweise eine Druckfeder sein, die die Wadenpelotte 20 in Figur 9 nach rechts drückt. Wird die Knieorthese gemäß Figur 9 folglich an ein Knie angelegt, drückt das Bein und insbesondere die Wade das elastische Lagerungselement 28 zusammen, sodass eine Kraft auf die Wadenpelotte 18 ausgeübt wird.

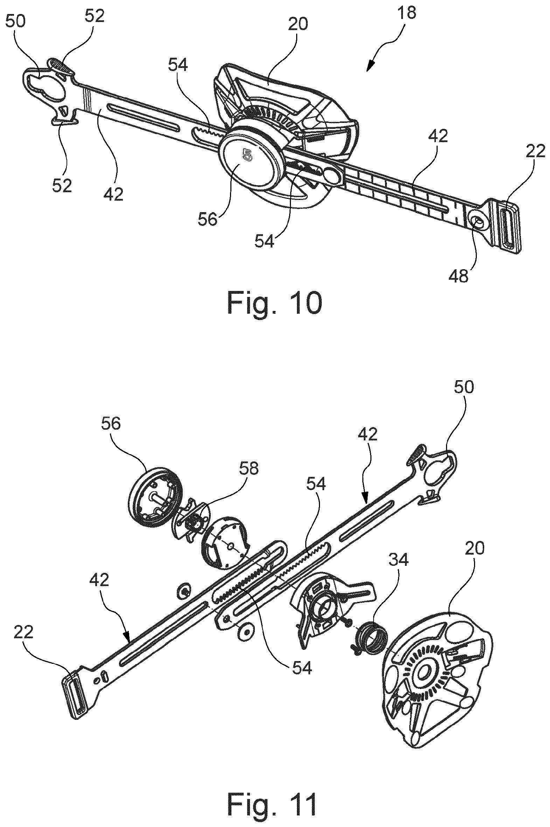

Figur 10 zeigt den Pelottenträger 18, der in den Orthesen gemäß Figuren 9 und 8 verwendet wird. Er verfügt über die beiden Arme 42, die seitlich von der Wadenpelotte 20 hervor stehen. An dem in Figur 10 rechts dargestellten Arm 42 befindet sich eine Ausnehmung 48, durch die ein Befestigungsstift 44 hindurchgeführt wird, um den Arm 42 am Unterschenkelrahmen 6 zu befestigen. Daneben befindet sich ein Verbindungselement 22, an dem beispielsweise ein Befestigungsgurt 32 angeordnet werden kann. Durch einen solchen nicht dargestellten Befestigungsgurt 32 kann eine weitere Stabilitätserhöhung erreicht werden und die Knieorthese sicherer am Bein des Trägers angeordnet werden.

Am in Figur 10 links dargestellten Arm 42 befindet sich ein Formschlusselement 50, das mit einem am Unterschenkelrahmen 6 angeordneten korrespondierend ausgebildeten Formschlusselement so zusammenwirken kann, dass der Arm 42 besonders leicht am Unterschenkelrahmen 6 angeordnet werden kann. Dabei kommt es zu einer formschlüssigen Verbindung der beiden Formschlusselemente. Soll die Knieorthese gemäß den Figuren 8 und 9 angelegt werden, wird bevorzugt das Formschlusselement 50 vom korrespondierend ausgebildeten Formschlusselement am Unterschenkelrahmen 6 gelöst, bevor die Knieorthese angelegt wird. Erst wenn der Oberschenkelrahmen 2 am Oberschenkel und der Unterschenkelrahmen 6 am Unterschenkel anliegt wird das Formschlusselement 50 mit dem korrespondierend ausgebildeten Formschlusselement am Unterschenkelrahmen 6 verbunden. Dazu verfügt das Ende des Arms 42 über Greifelemente 52, die es dem Träger der Orthese ermöglichen, das Ende des Arms 42 besonders leicht zu ergreifen und mit dem Formschlusselement am Unterschenkelrahmen 6 zu verbinden.

Die beiden Arme 42 sind mit jeweils einer Zahnreihe 54 ausgerüstet, die in ein nicht dargestellt des Formschlusselement eingreift. Es entsteht bevorzugt eine Rastverbindung zwischen jeweils einem der Arme 42 und einem der Formschlusselemente. In einer bevorzugten Ausgestaltung kann der Deckel 56 gedreht werden, um diese Rastverbindung zu lösen. In dieser Position ist wenigstens ein Arm 42, bevorzugt jeder Arm 42 relativ zur Wadenpelotte 20 verschiebbar, sodass die wirksame Länge des Pelottenträgers 18 eingestellt werden kann. Dadurch kann eingestellt werden, wie stark das nicht dargestellte elastische Lagerungselement 28 durch die Wade des Trägers komprimiert wird, sodass die auf die Wade wirkende Kraft einstellbar ist. Vorzugsweise handelt es sich bei der wirksamen Länge des Pelottenträgers 18 um den Abstand zwischen der Ausnehmung 48 und dem Formschlusselement 50.

Figur 11 zeigt das Bauteil aus Figur 10 in einer Explosion da. Man erkennt die beiden Arme 42, die jeweils eine Zahnreihe 54 aufweisen und die relativ zueinander verschiebbar ausgebildet sind. Zwischen den Armen 42 und dem deckelsechsundfünfzig findet sich unter anderem ein Zahnrad 58, dass mit den beiden Zahlenreihen 54 in Eingriff gebracht wird. Auf diese Weise wird eine einmal eingestellte Länge, die dem Abstand zwischen dem Verbindungselement 22 und dem Formschlusselement 50 entspricht arretiert. Die eigentliche Wadenpelotte 20 ist über die Spiralfeder 34 elastisch gelagert.

### Bezugszeichenliste

- 2: Oberschenkelrahmen
- 4: Gelenk
- 6: Unterschenkelrahmen
- 8: mediale Schiene
- 10: frontales Verbindungselement
- 12: Gurt
- 14: mediale Schiene
- 16: dorsales Verbindungselement
- 18: Pelottenträger
- 20: Wadenpelotte
- 22: Verbindungselement
- 24: Gurt
- 26: frontales Verbindungselement
- 28: elastisches Lagerungselement
- 30: Kissen
- 32: Befestigungsgurt
- 34: Spiralfeder
- 36: Führungsstift
- 38: Stiftaufnahme
- 40: Dehnungsbegrenzer
- 42: Arm
- 44: Befestigungsstift
- 46: Loch
- 48: Ausnehmung
- 50: Formschlusselement
- 52: Greifelement
- 54: Zahnreihe
- 56: Deckel
- 58: Zahnrad

## Patentansprüche

1. Knieorthese mit
- einem Oberschenkelrahmen (2) zum Anordnen an einem Oberschenkel und
- einem Unterschenkelrahmen (6) zum Anordnen an einem Unterschenkel,
o wobei der Unterschenkelrahmen (6) mittels wenigstens eines Gelenkes (4) um eine erste Schwenkachse schwenkbar an dem Oberschenkelrahmen (2) angeordnet ist, und
- einer Wadenpelotte (20) zum Anlegen an eine Wade,
wobei die Knieorthese wenigstens ein elastisches Lagerungselement (28) aufweist, das derart angebracht ist, dass das Lagerungselement (28) beim Anlegen der Knieorthese gespannt wird und in diesem Zustand die Wadenpelotte (20) mittelbar oder unmittelbar an eine Wade des Trägers der Knieorthese drückt und wobei die Knieorthese einen Pelottenträger (18) aufweist,
**dadurch gekennzeichnet, dass** H
der Pelottenträger (18) an dem Unterschenkelrahmen (6) angeordnet ist, wobei die Wadenpelotte (20) über das wenigstens eine elastische Lagerungselement (28) an dem Pelottenträger (18) angeordnet ist.

2. Knieorthese nach Anspruch 1, **dadurch gekennzeichnet, dass** das Lagerungselement (28) im gespannten Zustand eine nach frontal wirkende Kraft auf die Wadenpelotte (20) ausübt.

3. Knieorthese nach Anspruch 1 oder 2, **dadurch gekennzeichnet, dass** der Pelottenträger (18) in mehreren unterschiedlichen Positionen und/oder Orientierungen relativ zu dem Unterschenkelrahmen (6) an dem Unterschenkelrahmen (6) festlegbar ist.

4. Knieorthese nach Anspruch 3, **dadurch gekennzeichnet, dass** die Position und/oder die Orientierung des Pelottenträgers (18) relativ zu dem Unterschenkelrahmen (6) stufenlos einstellbar ist.

5. Knieorthese nach einem der vorstehenden Ansprüche, **dadurch gekennzeichnet, dass** die Wadenpelotte (20) lösbar an dem Pelottenträger (18) angeordnet ist.

6. Knieorthese nach einem der vorstehenden Ansprüche, **dadurch gekennzeichnet, dass** das wenigstens eine Lagerungselement (28) eine Kompressionselement, vorzugsweise eine Druckfeder und/oder ein Kompressionselement aus einem elastischen Material aufweist.

7. Knieorthese nach einem der vorstehenden Ansprüche, **dadurch gekennzeichnet, dass** das wenigstens eine Lagerungselement (28) wenigstens ein Zugelement, vorzugsweise eine Zugfeder und/oder wenigstens einen elastischen Gurt aufweist.

8. Knieorthese nach einem der vorstehenden Ansprüche, **dadurch gekennzeichnet, dass** die Wadenpelotte (20) um wenigstens eine zweite Schwenkachse schwenkbar an dem Pelottenträger (18) angeordnet ist, die vorzugsweise senkrecht auf die erste Schwenkachse steht.

9. Knieorthese nach einem der vorstehenden Ansprüche, **dadurch gekennzeichnet, dass** die Knieorthese eine Messskala aufweist, an der eine Stärke der Spannung des wenigstens einen elastischen Lagerungselementes (28) und/oder einer durch die Spannung hervorgerufene Kraft ablesbar ist.

## Claims

1. A knee orthosis with
- an upper leg frame (2) for arranging on an upper leg and
- a lower leg frame (6) for arranging on a lower leg,
o wherein the lower leg frame (6) is arranged on the upper leg frame (2) such that it can be swivelled about a first swivel axis by means of at least one joint (4), and
- a calf pad (20) for mounting on a calf,
wherein
the knee orthosis comprises at least one elastic bearing element (28) that is attached in such a way that the bearing element (28) is tensioned when the knee orthosis is mounted and, in this state, presses the calf pad (20) either directly or indirectly against a calf of the wearer of the knee orthosis and wherein the knee orthosis comprises a pad retainer (18), **characterized in that** the pad retainer (18) is arranged on the lower leg frame (6), wherein the calf pad (20) is arranged on the pad retainer (18) via the at least one elastic bearing element (28).

2. The knee orthosis according to claim 1, **characterized in that** the bearing element (28) in the tensioned state exerts a frontal-acting force on the calf pad (20).

3. The knee orthosis according to claim 1 or 2, **characterized in that** the pad retainer (18) can be fixed on the lower leg frame (6) in multiple positions and/or orientations relative to the lower leg frame (6).

4. The knee orthosis according to claim 3, **characterized in that** the position and/or orientation of the pad retainer (18) is infinitely adjustable relative to the lower leg frame (6).

5. The knee orthosis according to one of the preceding claims, **characterized in that** the calf pad (20) is detachably arranged on the pad retainer (18).

6. The knee orthosis according to one of the preceding claims, **characterized in that** the at least one bearing element (28) has a compression element, preferably a compression spring and/or a compression element made of an elastic material.

7. The knee orthosis according to one of the preceding claims, **characterized in that** the at least one bearing element (28) has at least one tension element, preferably a tension spring and/or at least one elastic strap.

8. The knee orthosis according to one of the preceding claims, **characterized in that** the calf pad (20) is arranged on the pad retainer (18) such that it can be swivelled about at least a second swivel axis, which is preferably orthogonal to the first swivel axis.

9. The knee orthosis according to one of the preceding claims, **characterized in that** the knee orthosis features a measurement scale on which a strength of the tension of the at least one elastic bearing element (28) and/or a force generated by this tension can be read.

## Revendications

1. Orthèse de genou, comprenant
- un cadre de cuisse (2) destiné à être placé sur une cuisse, et
- un cadre de jambe (6) destiné à être placé sur une jambe,
• le cadre de jambe (6) étant disposé sur le cadre de cuisse (2) de manière à pouvoir pivoter autour d'un premier axe de pivotement au moyen d'au moins une articulation (4), et
- une pelote de mollet (20) destinée à être appliquée contre un mollet,
dans laquelle
l'orthèse de genou présente au moins un élément de support élastique (28) qui est monté de telle sorte que l'élément de support (28) est tendu lors de l'application de l'orthèse de genou et que, dans cet état, la pelote de mollet (20) est pressée directement ou indirectement contre un mollet du porteur de l'orthèse de genou, et
l'orthèse de genou comprend un porte-pelote (18),
**caractérisée en ce que**
le porte-pelote (18) est disposé sur le cadre de jambe (6), la pelote de mollet (20) étant disposée sur le porte-pelote (18) par l'intermédiaire dudit au moins un élément de support élastique (28).

2. Orthèse de genou selon la revendication 1,
**caractérisée en ce que** l'élément de support (28) exerce, à l'état tendu, une force agissant vers l'avant sur la pelote de mollet (20).

3. Orthèse de genou selon la revendication 1 ou 2,
**caractérisée en ce que** le porte-pelote (18) peut être immobilisé sur le cadre de jambe (6) dans plusieurs positions et/ou orientations différentes par rapport au cadre de jambe (6).

4. Orthèse de genou selon la revendication 3,
**caractérisée en ce que** la position et/ou l'orientation du porte-pelote (18) par rapport au cadre de jambe (6) est réglable en continu.

5. Orthèse de genou selon l'une des revendications précédentes,
**caractérisée en ce que** la pelote de mollet (20) est montée de manière amovible sur le porte-pelote (18).

6. Orthèse de genou selon l'une des revendications précédentes,
**caractérisée en ce que** ledit au moins un élément de support (28) présente un élément de compression, de préférence un ressort de compression et/ou un élément de compression en matériau élastique.

7. Orthèse de genou selon l'une des revendications précédentes,
**caractérisée en ce que** ledit au moins un élément de support (28) présente au moins un élément de traction, de préférence un ressort de traction et/ou au moins une sangle élastique.

8. Orthèse de genou selon l'une des revendications précédentes,
**caractérisée en ce que** la pelote de mollet (20) est disposée sur le porte-pelote (18) de manière à pouvoir pivoter autour d'au moins un deuxième axe de pivotement, qui est de préférence perpendiculaire au premier axe de pivotement.

9. Orthèse de genou selon l'une des revendications précédentes,
**caractérisée en ce que** l'orthèse de genou présente une échelle de mesure sur laquelle peut être lue une intensité de la tension dudit au moins un élément de support élastique (28) et/ou une force provoquée par la tension.
